(19) Europäisches Patentamt
European Patent Office
Office européen des brevets

(11) Veröffentlichungsnummer: **0 469 399 B1**

# (12) EUROPÄISCHE PATENTSCHRIFT

(45) Veröffentlichungstag der Patentschrift: **14.12.94**

(51) Int. Cl.[5]: **C07C 309/52**, C07C 225/22

(21) Anmeldenummer: **91112002.0**

(22) Anmeldetag: **18.07.91**

## (54) Aminobenzophenonsulfonsäuren und deren Zwischenprodukte.

(30) Priorität: **30.07.90 DE 4024120**

(43) Veröffentlichungstag der Anmeldung:
**05.02.92 Patentblatt 92/06**

(45) Bekanntmachung des Hinweises auf die Patenterteilung:
**14.12.94 Patentblatt 94/50**

(84) Benannte Vertragsstaaten:
**BE CH DE ES FR GB IT LI NL**

(56) Entgegenhaltungen:
**DE-A- 2 223 622**
**FR-A- 2 505 854**

**JOURNAL OF ORGANIC CHEMISTRY. Bd. 40, Nr. 8, 18. April 1975, EASTON US Seiten 1090 - 1094; J.R. PRATT ET AL: 'ORGANOSILICON COMPOUNDS.XX. SYNTHESIS OF AROMATIC DIAMINES VIA TRIMETHYLSILYL-PROTECTING ANILINE INTERMEDIATES'**

**JOURNAL OF APPLIED POLYMER SCIENCE. Bd. 26, Nr. 11, November 1981, NEW YORK US Seiten 3805 - 3817; V.L.BELL ET AL.: 'POLYIMIDE STRUCTURE-PROPERTY RELATIONSHIPS. III. POLYIMIDES FROM MULTI-RING DIAMINES'**

**JOURNAL OF CHROMATOGRAPHY Bd. 119, 1976, AMSTERDAM Seiten 569 - 579; P.R. YOUNG: 'HIGH-PRESURE LIOUID CHROMATOGRAPHY OF AROMATIC AMINES'**

**CHEMICAL ABSTRACTS, vol. 112, no. 22, 28. Mai 1990, Columbus, Ohio, US; abstract no. 200236Z, Seite 78 ;**

**CHEMICAL ABSTRACTS, vol. 111, no. 23, 4. Dezember 1989, Columbus, Ohio, US; abstract no. 214163, Seite 559 ;**

(73) Patentinhaber: **BASF Aktiengesellschaft**
**Carl-Bosch-Strasse 38**
**D-67063 Ludwigshafen (DE)**

(72) Erfinder: **Lamm, Gunther**
**Heinrich-Heine-Strasse 7**
**W-6733 Hassloch (DE)**
Erfinder: **Teich, Friedhelm**
**Luisenstrasse 2 a**
**W-7500 Karlsruhe 1 (DE)**

**EP 0 469 399 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Aminobenzophenone der Formel Ia

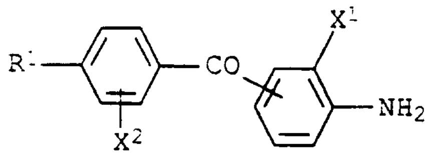

(Ia),

in der einer der Reste $X^1$ und $X^2$ Wasserstoff und der andere Hydroxysulfonyl und $R^1$ Chlor, $C_1$-$C_4$-Alkyl, Methoxy oder Ethoxy oder ein Rest der Formel

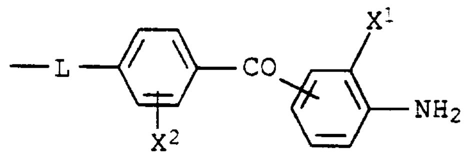

bedeuten, worin L für eine chemische Bindung, $C_1$-$C_4$-Alkylen oder den Rest der Formel -O-$CH_2$- steht und $X^1$ und $X^2$ jeweils die obengenannte Bedeutung besitzen, und worin die Carbonylgruppe jeweils in para-Position zu $NH_2$ oder $X^1$ steht, sowie neue Aminobenzyphenone der Formel Ib oder Ic

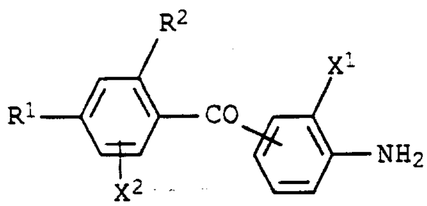

(Ib)

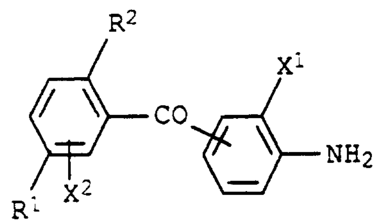

(Ic),

in der einer der Reste $X^1$ und $X^2$ Wasserstoff und der andere Hydroxysulonyl und $R^1$ und $R^2$ unabhängig voneinander Chlor, $C_1$-$C_4$-Alkyl, Methoxy oder Ethoxy bedeuten und wobei die Carbonylgruppe jeweils in para-Position zu $NH_2$ oder $X^1$ steht.

Die neuen Aminobenzophenone der Formel Ia bis Ic sind in Form der freien Säure angegeben. Selbstverständlich werden jedoch auch ihre Salze mit umfaßt.

Als Salze kommen dabei Metall- oder Ammoniumsalze in Betracht. Metallsalze sind insbesondere die Lithium-, Natrium- oder Kaliumsalze. Unter Ammoniumsalzen im erfindungsgemäßen Sinne sind solche Salze zu verstehen, die entweder unsubstituierte oder substituierte Ammoniumkationen aufweisen. Substituierte Ammoniumkationen sind z.B. Monoalkyl-, Dialkyl-, Trialkyl-, Tetraalkyl- oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium-, Piperazinium- oder N-Alkylpiperaziniumkationen oder deren N-monoalkyl- oder N,N-dialkylsubstituierte Produkte. Unter Alkyl ist dabei im allgemeinen geradkettiges oder verzweigtes $C_1$-$C_{20}$-Alkyl zu verstehen, das durch Hydroxylgruppen substituiert und/oder durch Sauerstoffatome in Etherfunktion unterbrochen sein kann.

Aus der EP-A-302 401 sind Aminobenzophenone bekannt, die jedoch keinen Säurerest im Molekül aufweisen. Weiterhin beschreibt die DE-A-2 223 622 einen Azofarbstoff, dessen Diazokomponente 3-Amino-4-hydroxysulfonylbenzophenon ist.

Aufgabe der vorliegenden Erfindung war es nun, neue Aminobenzophenone bereitzustellen, die über mindestens einen Hydroxysulfonylrest verfügen.

Demgemäß wurden die eingangs näher bezeichneten Aminobenzophenone der Formel Ia bis Ic gefunden.

Alle in der obengenannten Formel I auftretenden Alkyl- und Alkylengruppen können sowohl geradkettig als auch verzweigt sein.

Reste $R^1$ und $R^2$ sind z.B. Methyl, Ethyl, Propyl, Isopropyl, Butyl, Isobutyl oder sec-Butyl.

Reste L sind z.B. Methylen, Ethylen, 1,2- oder 1,3-Propylen, Isopropyliden oder 1,2-, 1,3-, 2,3- oder 1,4-Butylen.

Wenn $R^1$ und $R^2$ in den obengenannten Formeln $C_1$-$C_4$-Alkyl bedeutet, sind solche Aminobenzophenone bevorzugt, in denen $R^1$ und $R^2$ Methyl oder Ethyl bedeuten.

Die erfindungsgemäßen Aminobenzophenone der Formel Ia bis Ic können nach an sich bekannten Methoden erhalten werden.

Beispielsweise kann man ein geeignetes Benzophenonderivat, das frei ist von Sulfonsäuregruppen ($X^1$ und $X^2$ in Formel Ia, Ib und Ic sind jeweils Wasserstoff) und das entweder schon die Aminogruppe oder zunächst eine Nitrogruppe aufweist, auf übliche Weise mit einem geeigneten Sulfonierungsmittel behandeln.

Geeignete Sulfonierungsmittel sind z.B. Schwefelsäure, Chlorsulfonsäure, Oleum oder deren Mischungen.

Je Moläquivalent des einzuführenden Hydroxysulfonylrests wendet man in der Regel 1 Mol an Sulfonierungsmittel an.

Die Sulfonierung wird im allgemeinen bei einer Temperatur von 20 bis 200 °C, vorzugsweise 50 bis 180 °C, vorgenommen.

Man kann dabei entweder in Anwesenheit oder Abwesenheit von einem Lösungsmittel arbeiten. Geeignete Lösungsmittel sind z.B. inerte organische Lösungsmittel, wie o-Dichlorbenzol oder Trichlorbenzol, oder auch überschüssiges Sulfonierungsmittel.

Bei der Sulfonierung von Benzophenonderivaten, die eine Aminogruppe aufweisen, führt man die Sulfonierung vorzugsweise in Abwesenheit eines inerten organischen Lösungsmittels jedoch mit einem geringen überschuß an Sulfonierungsmittel (ca. 0,01 bis 0,3 Mol, bezogen auf 1 Mol Benzophenon III) durch.

Nach beendeter Sulfonierung erfolgt die Aufarbeitung nach an sich bekannten Methoden. Beispielsweise kann man, gegebenenfalls nach Entfernung des inerten organischen Lösungsmittels mittels Wasserdampfdestillation, den Rückstand in Wasser lösen und bei einem pH-wert, der in der Regel kleiner als 1 ist, z.B. mittels Natriumchlorid aussalzen.

Führt man die Sulfonierung an Benzophenonderivaten, die eine Nitrogruppe aufweisen, durch, so muß anschließend noch die Nitrogruppe durch Reduktion in die Aminogruppe übergeführt werden. Dies gelingt auf an sich bekanntem Wege, z.B. mittels Wasserstoff in Gegenwart eines Katalysators. Geeignete Katalysatoren sind z.B. Raney-Nickel oder Palladium auf einem Kohleträger.

Die Herstellung der Benzophenonderivate, die frei sind von Sulfonsäuregruppen, erfolgt ebenfalls nach an sich bekannten Methoden. Beispielsweise kann man ein Benzoylchlorid der Formel II

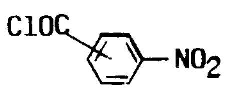 (II),

unter den Reaktionsbedingungen einer Friedel-Crafts-Acylierung mit einem Aromaten der Formel IIIa, IIIb oder IIIc

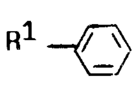 (IIIa) 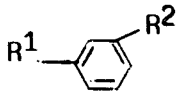 (IIIb) 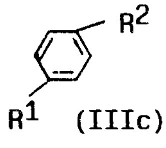 (IIIc)

worin $R^1$ und $R^2$ jeweils die obengenannte Bedeutung besitzen, umsetzen und das resultierende Benzophenon gegebenenfalls reduzieren.

Die erfindungsgemäßen Aminobenzophenone der Formel Ia bis Ic sind wertvolle Zwischenprodukte für die Synthese von Farbstoffen oder Wirkstoffen.

Die folgenden Beispiele sollen die Erfindung näher erläutern.

Beispiel 1

105,5 g 4-Amino-4'-methylbenzophenon wurden in 500 ml o-Dichlorbenzol suspendiert. Dann versetzte man das Gemisch mit 62 g Schwefelsäure (96 gew.-%ig) und erhitzte auf Siedekühlung (175 bis 180 °C). Dabei wurde das zurückgeführte o-Dichlorbenzol über einen Wasserabscheider geleitet, in dem das Reaktionswasser sowie das in der Schwefelsäure befindliche Wasser abgetrennt wurden. Nach ca. 1 Stunde trennte sich kein Wasser mehr ab. Dann leitete man das wasserfeuchte o-Dichlorbenzol-Kondensat über trockenes Kieselgel. (Es kann auch durch frisches, trockenes o-Dichlorbenzol ersetzt werden). Auf diese Weise gelang die Umsetzung zu 4-Amino-4'-methylbenzophenon-3-sulfonsäure

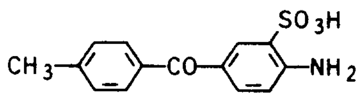

praktisch quantitativ.

Nach beendeter Umsetzung (ca. 5 bis 7 Stunden) wurde o-Dichlorbenzol durch Wasserdampfdestillation abgetrennt. Der Rückstand wurde mit 50 gew.-%iger Natronlauge in Wasser gelöst und die Lösung durch Zugabe von 1 g Aktivkohle klärfiltriert. Das Filtrat wurde mit konz. Salzsäure auf einen pH-Wert, der kleiner als 1 war, gestellt und auf Raumtemperatur abgekühlt. Nach Absaugen, Waschen mit wenig stark verdünnter Salzsäure und Trocknen erhielt man 120 g 4-Amino-4'-methylbenzophenon-3-sulfonsäure.
Schmelzpunkt: 280 °C (Zers.)
$\lambda_{max}$ (gemessen in N,N-Dimethylformamid): 332 nm

Beispiel 2

241 g 4-Methyl-4'-nitrobenzophenon wurden bei Raumtemperatur in 700 g Oleum (24 gew.-%ig) eingetragen. Dann hob man die Temperatur des Gemisches auf 65 °C an und rührte 5 Stunden bei dieser Temperatur nach. Die Reaktion war danach beendet. Man kühlte auf Raumtemperatur ab und fällte auf 1600 ml Eiswasser, gab 150 g Natriumchlorid hinzu und kühlte auf Raumtemperatur ab. Das ausgefallene Produkt der Formel

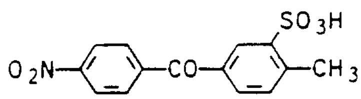

wurde abgesaugt, mit wenig eiskalter, verdünnter Salzsäure gewaschen und getrocknet. Man erhielt 240 g 4-Methyl-4'-nitrobenzophenon-3-sulfonsäure. Dieses Produkt wurde dann mit 1500 ml Methanol und 100 ml Wasser angerührt, mit 50 gew.-%iger Natronlauge auf einen pH-Wert von 5,5 gestellt und dann mit Wasserstoff in Gegenwart von 3 g fein verteiltem Raney-Nickel bei 40 bis 50 °C und Normaldruck hydriert.

Nach Abtrennung des Nickels und Abdestillieren des Methanols säuerte man auf einen pH-Wert von ca. 0,5 an und isolierte die Aminoverbindung der Formel

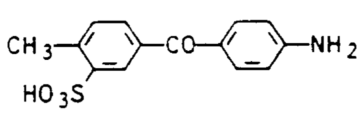

durch Aussalzen, wie in Beispiel 1 beschrieben.
Ausbeute: 208 g, Schmelzpunkt: >300 °C; $\lambda_{max}$ (gemessen in Wasser): 336, 252 nm

Beispiel 3

Man arbeitete analog Beispiel 1, verwendete aber als Benzophenon 105,5 g des Amins der Formel

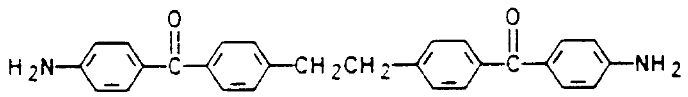

(Schmelzpunkt: 220°C)

sowie 74 g konzentrierte Schwefelsäure. Man erhielt ein Produkt der Formel

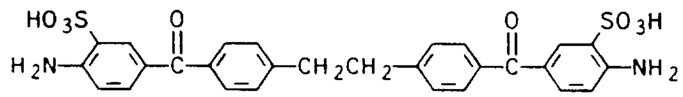

$\lambda_{max}$ (gemessen in N,N-Dimethylformamid): 331 nm

Beispiel 4

4,78 kg 4-Amino-4'-isopropylbenzophenon wurden bei 120 °C innerhalb von 30 Minuten unter Rühren mit 2100 g Schwefelsäure (96 gew.-%ig) versetzt. Man erhielt ein gut rührbares, homogenes Gemisch, das innerhalb von 3 Stunden auf 165 bis 175 °C aufgeheizt und im Backverfahren bei dieser Temperatur unter Stickstoffspülung zu 4-Amino-4'-isopropylbenzophenon-3-sulfonsäure der Formel

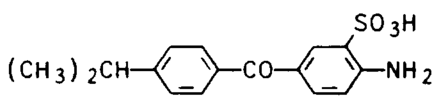

umgesetzt wurde.

Die Umsetzung erfolgte quantitativ. (Auch bei Verwendung stöchiometrischer Mengen Schwefelsäure (2050 g) fand man nur Spuren von nicht sulfoniertem Ausgangsprodukt).

Man erhielt ein graues Pulver, das sich in Wasser in Gegenwart von Natronlauge rückstandsfrei löste.

$\lambda_{max}$ (gemessen in N,N-Dimethylformamid): 332 nm, Ausbeute: 6,76 kg. (Das Produkt enthielt noch 416 g Schwefelsäure).

In analoger Weise können die in den Tabellen 1 und 2 aufgeführten Benzophenonderivate erhalten werden.

Tabelle 1

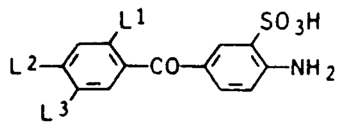

| Bsp. Nr. | $L^1$ | $L^2$ | $L^3$ |
|---|---|---|---|
| 5 | $CH_3$ | $CH_3$ | H |
| 6 | H | Cl | H |
| 7 | $CH_3$ | H | $CH_3$ |
| 8 | Cl | $CH_3$ | H |
| 9 | $OCH_3$ | $CH_3$ | H |

Tabelle 2

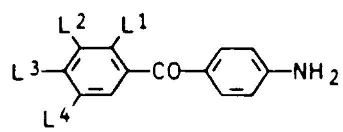

| Bsp. Nr. | $L^1$ | $L^2$ | $L^3$ | $L^4$ |
|---|---|---|---|---|
| 10 | $CH_3$ | $SO_3H$ | H | $CH_3$ |
| 11 | $OCH_3$ | $SO_3H$ | H | $CH_3$ |
| 12 | $CH_3$ | H | $OCH_3$ | $SO_3H$ |
| 13 | H | H | Cl | $SO_3H$ |
| 14 | H | H | $C_2H_5$ | $SO_3H$ |
| 15 | H | H | $C_4H_9(n)$ | $SO_3H$ |

Beispiel 16

251 g 2,4-Dimethyl-3'-nitrobenzophenon wurden analog Beispiel 2 in Oleum sulfoniert und anschließend zur Verbindung der Formel

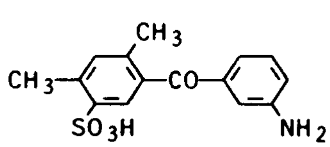

$\lambda_{max}$ (gemessen in Wasser bei pH ≥ 7): 328 nm

reduziert.

In analoger Weise können auch die folgenden Komponenten erhalten werden.

**17**

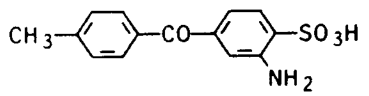

$\lambda_{max}$ (gemessen in Wasser bei pH 7): 238 nm,
268 nm
325 nm (niedrig)

**18**

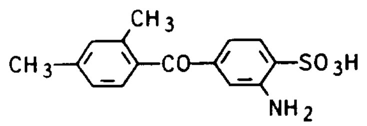

$\lambda_{max}$ (gemessen in Wasser bei pH 7): 237 nm,
267 nm

**19**

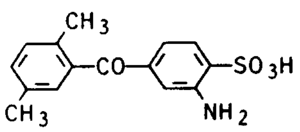

Fp. > 300°C

20

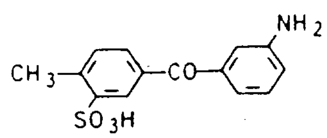

$\lambda_{max}$ (gemessen in Wasser bei pH ≥7): 263 nm,
328 nm,
2-6 nm

21

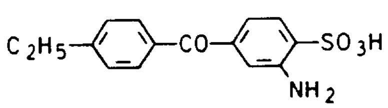

$\lambda_{max}$ (gemessen in Wasser bei pH 7): 239 nm,
268 nm,
326 nm

22

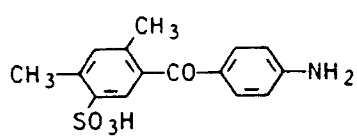

$\lambda_{max}$ (gemessen in Wasser: 336 nm)
Fp.: 330°C (Zers.)

23

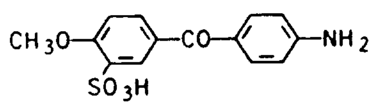

$\lambda_{max}$ (gemessen in Wasser bei pH 7): 330 nm

Beispiel 24 (Vorstufe für Beispiel 3)

In 2500 ml Methylenchlorid (wasserfrei) wurden bei Raumtemperatur 480 g trockenes Aluminiumchloridpulver und danach portionsweise 600 g p-Nitrobenzoylchlorid gegeben. Anschließend wurde das Gemisch mit 274 g 1,2-Diphenylethan (wasserfrei) versetzt und über Nacht bei 35 °C gerührt. Danch zerlegte man den Komplex auf Wasser, trennte die obere, wäßrige Phase ab, extrahierte die organische Phase zweimal

mit Wasser (pH ca. 7) und destillierte dann das organische Lösungsmittel ab. Nach Absaugen, Waschen und Trocknen des Rückstandes erhielt man 680 g der verbindung der Formel

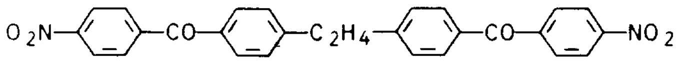

(Schmelzpunkt 200°C).

360 g dieses Produktes wurden in 2500 ml N,N-Dimethylformamid und 30 ml Eisessig mit Wasserstoff mit Hilfe eines Nickelkatalysators bei 80 °C hydriert. Man trennte nach beendeter Wasserstoffaufnahme vom Katalysator ab und fällte das Diamin der Formel

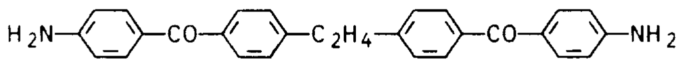

(Schmelzpunkt 220°C).

mit Wasser aus. Man erhielt 310 g des Produktes.

**Patentansprüche**

**1.** Aminobenzophenone der Formel Ia

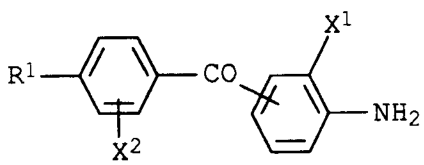

(Ia),

in der einer der Reste $X^1$ und $X^2$ Wasserstoff und der andere Hydroxysulfonyl und $R^1$ Chlor, $C_1$-$C_4$-Alkyl, Methoxy oder Ethoxy oder ein Rest der Formel

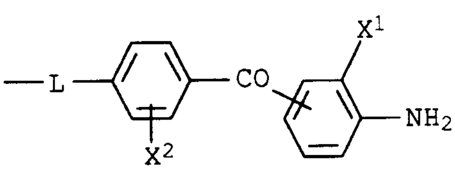

bedeuten, worin L für eine chemische Bindung, $C_1$-$C_4$-Alkylen oder den Rest der Formel -O-$CH_2$- steht und $X^1$ und $X^2$ jeweils die obengenannte Bedeutung besitzen, und worin die Carbonylgruppe jeweils in para-Position zu $NH_2$ oder $X^1$ steht.

**2.** Aminobenzophenone der Formel Ib oder Ic

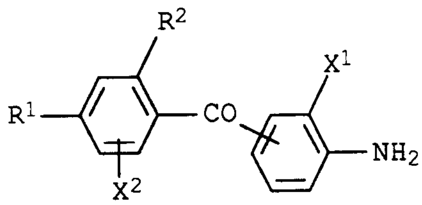

(Ib)

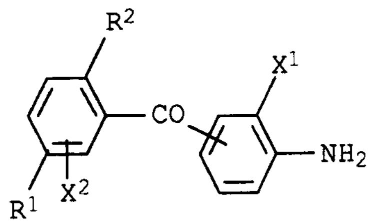

(Ic),

in der einer der Reste $X^1$ und $X^2$ Wasserstoff und der andere Hydroxysulfonyl und $R^1$ und $R^2$ unabhängig voneinander Chlor, $C_1$-$C_4$-Alkyl, Methoxy oder Ethoxy bedeuten und wobei die Carbonylgruppe jeweils in para-Position zu $NH_2$ oder $X^1$ steht.

**3.** Aminobenzophenone nach Anspruch 1, dadurch gekennzeichnet, daß einer der beiden Reste $X^1$ und $X^2$ Wasserstoff und der andere Hydroxysulfonyl bedeuten.

**4.** Verdoppelte Aminobenzophenone der Formel II

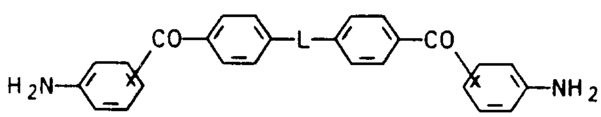

(II),

in der
L eine chemische Bindung, $C_1$-$C_4$-Alkylen oder der Rest der Formel -O-$CH_2$- bedeutet.

**Claims**

**1.** Aminobenzophenones of the formula Ia

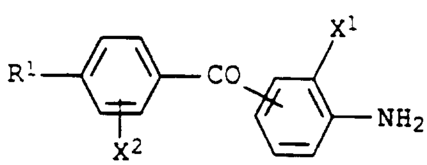

**(Ia)**

where one of $X^1$ and $X^2$ is hydrogen and the other is hydroxysulfonyl and $R^1$ is chlorine, $C_1$-$C_4$-alkyl, methoxy or ethoxy or a radical of the formula

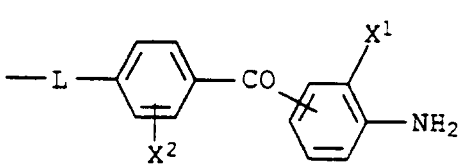

where L is a chemical bond, $C_1$-$C_4$-alkylene or a radical of the formula -O-$CH_2$-, $X^1$ and $X^2$ are each as

defined above, and the carbonyl group is para to $NH_2$ or $X^1$.

**2.** Aminobenzophenones of the formula Ib or Ic

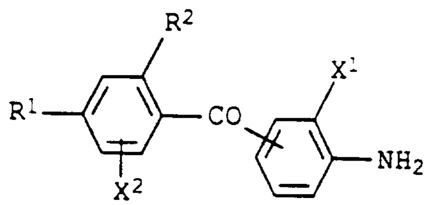

(Ib)

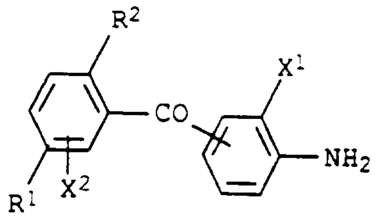

(Ic)

where one of $X^1$ and $X^2$ is hydrogen and the other is hydroxysulfonyl and $R^1$ and $R^2$ are each independently of the other chlorine, $C_1$-$C_4$-alkyl, methoxy or ethoxy, and the carbonyl group is in either case para to $NH_2$ or $X^1$.

**3.** Aminobenzophenones as claimed in claim 1, wherein one of $X^1$ and $X^2$ is hydrogen and the other is hydroxysulfonyl.

**4.** Doubled aminobenzophenones of the formula II

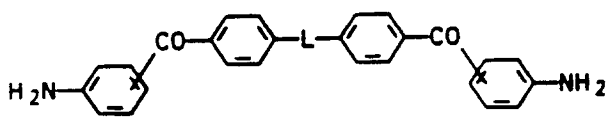

(II)

where
L is a chemical bond, $C_1$-$C_4$-alkylene or a radical of the formula $-O-CH_2-$.

**Revendications**

**1.** Aminobenzophénones de formule Ia

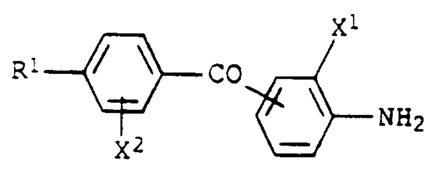

(Ia),

dans laquelle l'un des restes $X^1$ et $X^2$ représente un atome d'hydrogène et l'autre un groupe hydroxysulfonyle et $R^1$ représente un atome de chlore, un groupe alkyle en $C_1$-$C_4$, méthoxy ou éthoxy ou un reste de formule

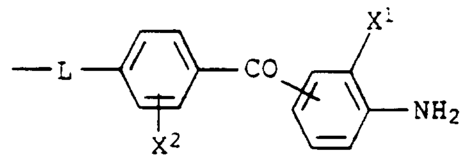

dans laquelle L est mis pour une liaison chimique, un groupe alkylène en $C_1$-$C_4$ ou le reste de formule -O-$CH_2$-et $X^1$ et $X^2$ ont chacun la signification mentionnée ci-dessus, et dans laquelle le groupe carbonyle est chaque fois en position para par rapport a $NH_2$ ou $X^1$.

**2.** Aminobenzophénones de formule Ib ou Ic

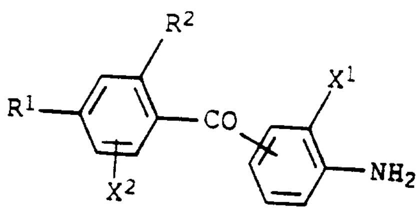

(Ib)

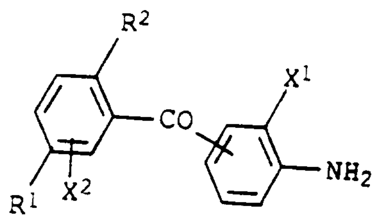

(Ic),

dans lesquelles l'un des restes $X^1$ et $X^2$ représente un atome d'hydrogène et l'autre un groupe hydroxysulfonyle et $R^1$ et $R^2$ représentent indépendamment l'un de l'autre un atome de chlore, un groupe alkyle en $C_1$-$C_4$, méthoxy ou éthoxy et dans lesquelles le groupe carbonyle est chaque fois en position para par rapport à $NH_2$ ou $X^1$.

**3.** Aminobenzophénones selon la revendication 1, caractérisées en ce que l'un des deux restes $X^1$ et $X^2$ représente un atome d'hydrogène et l'autre un groupe hydroxysulfonyle.

**4.** Aminobenzophénones doublées de formule II

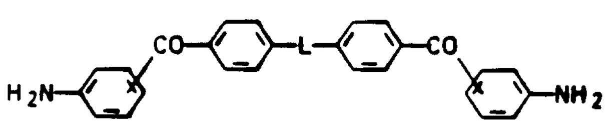

(II),

dans laquelle
L représente une liaison chimique, un groupe alkylène en $C_1$-$C_4$ ou le reste de formule -O-$CH_2$.